# EUROPÄISCHE PATENTSCHRIFT

(11) **EP 0 846 463 B1**
(45) Veröffentlichungstag und Bekanntmachung des Hinweises auf die Patenterteilung: **26.02.2003**
(21) Anmeldenummer: 97119880.9
(22) Anmeldetag: 13.11.1997
(51) Int. Cl.: A61K 9/70, A61L 15/58

(54) **Wirkstoffhaltige Pflaster**
Active substance containing plaster
Pansement adhésif contenant un médicament

(30) Priorität: 05.12.1996 DE 19650471
(43) Veröffentlichungstag der Anmeldung: 10.06.1998
(73) Patentinhaber: Beiersdorf Aktiengesellschaft, 20245 Hamburg (DE)
(72) Erfinder: Himmelsbach, Peter, 21614 Buxtehude (DE); Linder, Helmut, Dr., 22459 Hamburg (DE); Broschk, Katharina, 20253 Hamburg (DE); Sinnen, Marike, 25421 Pinneberg (DE); Wasner, Matthias, 21029 Hamburg (DE)

(56) Entgegenhaltungen:
- EP-A- 0 305 756
- DE-C- 4 224 325
- US-A- 5 306 503

## Beschreibung

Die Erfindung betrifft wirkstoffhaltige Pflaster mit einem Trägermaterial und einer darauf aufgebrachten Heißschmelzselbstklebemasse, welche den Wirkstoff oder gegebenenfalls auch mehrere Wirkstoffe enthält, die an die Haut abgegeben werden.

Transdermale Therapeutische Systeme (TTS) sind Darreichungsformen von Medikamenten, die einen oder mehrere Arzneistoffe über einen definierten Zeitraum an ihrem Anwendungsort an die Haut abgeben. Es wird dabei zwischen systemisch und lokal wirksamen Darreichungsformen unterschieden. Bei systemisch wirkenden Darreichungsformen gelangt der Wirkstoff durch Diffusion durch die Haut in den Blutkreislauf und kann im ganzen Körper wirken. Lokal wirkende Darreichungsformen wirken dagegen nur an den applizierten Stellen. Der Wirkstoff verbleibt in der Haut bzw. in den darunter liegenden Schichten.

Es sind bereits zahlreiche Ausführungsformen derartiger Pflaster beschrieben worden, die z. Teil nach dem Reservoirprinzip arbeiten, bei welchem der Wirkstoff beispielsweise über eine Membran abgegeben wird, teilweise auch mit einem Matrixsystem oder mit komplizierterem mehrschichtigen Aufbau.

Es ist auch bekannt, daß die Klebemasse des Pflasters als den Wirkstoff enthaltende Matrix eingesetzt werden kann. Neben aus Lösung aufgetragenen Selbstklebemassen wurden auch schon Heißschmelzselbstklebemassen dafür vorgeschlagen, z.B. in der EP-A 663 431, EP-A 452 034, EP-A 305 757, DE-A 43 10 012, DE-A 42 22 334 und DE-C 42 24 325. Als Wirkstoffe wurden hierbei, wenn diese benannt wurden, systemisch wirkende aufgeführt.

Durchblutungsfördernde Wirkstoffpflaster gehören zu der Gruppe der lokal wirksamen therapeutischen Systeme. Die Anwendung solcher Pflaster ist angezeigt zur Behandlung von rheumatischen Beschwerden, Ischias, Hexenschuß, Nackensteifigkeit, Schulter-Arm-Schmerzen sowie Muskelverspannungen und -zerrungen, Muskelkater oder Muskel-, Gelenk- und Nervenschmerzen im Bereich des Bewegungsapparates.

Capsaicin, Belladonna und Nonivamid sind bekannte Wirkstoffe solcher lokal, durchblutungsfördernd wirkender Pflaster. Aufgrund ihrer Anwendung am Bewegungsapparat müssen sie in der Regel stark kleben. Üblicherweise werden die Pflaster vollflächig mit einer Harz-Kautschuk-Klebemasse beschichtet, welche den Wirkstoff enthält.

Derartige Pflaster, welche meist größer flächig aufgebracht werden müssen, zeigen jedoch nach dem Ablösen bei empfindlichen Patienten z. Teil deutliche mechanische Hautirritationen. Fallweise kommt es zu allergischen Reaktionen. Ihr Entfernen ist nach längerer Tragezeit bis zu einem gewissen Grade schmerzhaft.

Ein weiterer Nachteil der bekannten wärmewirksamen Pflaster mit einer Klebemasse auf Basis von natürlichem Kautschuk, die in Form einer Lösung mit organischen Lösungsmitteln auf den Pflasterträger aufgetragen wird, ist die vergleichsweise niedrige Freisetzungsrate des Wirkstoffs.

Aufgabe der Erfindung war es deshalb, durchblutungsfördernde (hyperämisierende) und damit wärmewirksame Pflaster zu entwickeln, welche sich durch eine gute Wirksamkeit, d.h. eine relativ hohe Freisetzungsrate, und gute Hautverträglichkeit bei gleichzeitig gutem Klebeverhalten auszeichnen sollten. Auch sollten sie sich technisch wenig aufwendig und umweltverträglich herstellen lassen.

Gelöst wird diese Aufgabe durch wirkstoffhaltige Pflaster gemäß Anspruch 1.

Es hat sich gezeigt, daß Heißschmelzselbstklebemassen sich für die Freisetzung von hyperämisierend und nur lokal wirkenden Substanzen eignen.

Diese Wirkstoffe sind in erster Linie die bekannten Inhaltsstoffe des Cayenne Pfeffers als auch das synthetische Capsacinoid NVA (Nonivamid) sowie Nicotinsäurederivate, bevorzugt Bencylnicotinat oder Propylnicotinat.

Die Mengenkonzentrationen liegen zwischen 0,01 bis ca. 20 Gew.-%, vorzugsweise 0,1 bis 10 Gew.-%.

Die Klebemasse basiert A-B auf A-B-A -Blockcopolymeren. Die harte Phase A ist vornehmlich Polystyrol oder dessen Derivate und die weiche Phase B enthält Ethylen, Propylen, Butylen, Butadien, Isopren oder deren Mischungen, hierbei besonders bevorzugt Ethylen und Butylen oder deren Mischungen.

Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 5% und 30%, da ein niedrigerer Styrolanteil die Klebemasse anschmiegsamer macht.

Insbesondere die gezielte Abmischung von A-B und A-B-A copolymeren ist vorteilhaft, wobei ein Anteil an A-B-copolymeren von kleiner 80 Gew.-% bevorzugt wird.

In einer vorteilhaften Ausführung weist die Haftschmelzklebemasse die nachfolgend angegebene Zusammensetzung auf:

| | |
|---|---|
| 10 bis 90 Gew.-% | Blockcopolymere A-B/A-B-A, |
| 5 bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher |
| weniger als 15 Gew.-% | Additive |
| weniger als 5 Gew.-% | Stabilisatoren |
| 0,1- 10 Gew.-% | Wirkstoff oder Wirkstoffe. |

In einer besonders bevorzugten Ausführungsform einer derartigen, zusätzlich mit dem hyperämisierenden Wirkstoff versehenen Klebemase ist diese auf einem A-B/A-B-A Blockcopolymer aus Styrol, Äthylen und Butylen aufgebaut mit einem Styrolanteil von weniger als 35 Gew.-%, und sie enthält 0,1 - 10 Gew.-% Nonivamid, bevorzugt etwa 0,2 Gew.-%.

Die Kohlenwasserstofföle, -wachse und -harze dienen als Klebrigmacher. Wobei sich die Öle, wie Paraffinkohlenwasserstofföle, oder Wachse, wie Paraffinkohlenwasserstoffwachse, durch ihre Konsistenz günstig auf die Hautverklebung auswirken. Als Weichmacher finden langkettige Fettsäuren und/oder deren Ester Verwendung. Diese Zusätze dienen dabei der Einstellung der Klebeeigenschaften und der Stabilität. Auch der Zusatz von mineralischen Füllstoffen in geringen Mengen ist möglich.

Die Klebemassen sind so eingestellt, daß sie bei einer Frequenz von 0,1 rad/s eine dynamisch-komplexe Glasübergangstemperatur von weniger als +10°C, bevorzugt von -3° bis -30°C, ganz besonders bevorzugt von -6° bis -25°C, aufweisen.

Durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Selbstklebemasse wird durch die hohe Kohäsivität des Polymeren erreicht. Die gute Anfaßklebrigkeit ergibt sich durch die eingesetzten Klebrigmacher und Weichmacher.

Die Verteilung der Wirkstoffe in der Klebemasse erfolgt in einem Thermohomogenisator, wie z.B. Thermomixer, Thermokneter, Walzenwerke oder Schneckensysteme. Die Zugabe des Wirkstoffs kann in die vollständig hergestellte Klebemasse erfolgen. Beispielhaft kann der Wirkstoff auch in eine Zwischenstufe oder in die Ausgangsmischung eingearbeitet werden.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet. Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur der Schmelzhaftkleber zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient (Q = tan δ) zwischen dem Verlustmodul (G" viskoser Anteil) und dem Speichermodul (G' elastischer Anteil) ermittelt: Q = tan δ = G""7G'.

Für das subjektive Empfinden der Anfaßklebrigkeit (Tack) wird eine hohe Frequenz gewählt, für die Scherfestigkeit eine niedrige Frequenz. Ein hoher Zahlenwert bedeutet eine bessere Anfaßklebrigkeit und eine schlechtere Scherstabilität.

Der komplexe-dynamische Glasübergangspunkt ist der Übergangspunkt vom amorphen in den viskoelastischen Bereich. Er entspricht dem Maximum der Temperaturfunktion bei vorgegebener Frequenz.

Bevorzugt werden erfindungsgemäß Haftschmelzklebemassen, bei denen das Verhältnis des viskosen Anteils zum elastischen Anteil bei einer Frequenz von 100 rad/s bei 25°C größer 0,7 ist oder Haftschmelzklebemassen, bei denen das Verhältnis des viskosen Anteils zum elastischen Anteil bei einer Frequenz von 0,1 rad/s bei 25°C kleiner 0,4 ist.

Die wirkstoffhaltige Klebemasse kann ganzflächig auf ein geeignetes anschmiegsames Trägermaterial, wie Gewebe, Gewirke, Folien, Vlies, Papier, Schaumstoff oder Laminate davon, aufgetragen werden, vorteilhaft kann sie auch flächenmäßig begrenzt beispielsweise durch Rasterdruck, Thermosiebdruck. Thermoflexodruck oder Tiefdruck aufgebracht werden. Dies führt zu einer besonders guten Luft- und Wasserdampfdurchlässigkeit der Klebeschicht.

Bevorzugt wird beim Siebdruck der Auftrag in Form von Kalotten, besonders solcher Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.

Ferner kann die Klebemasse beispielsweise auch aufgesprüht werden, was ein mehr oder weniger unregelmäßiges Auftragsbild ergibt.

Die Selbstklebemasse kann gleichmäßig auf dem Trägermaterial verteilt sein, sie kann aber auch funktionsgerecht für das Produkt über die Fläche unterschiedlich stark oder dicht aufgetragen sein.

Das Prinzip des Thermosiebdrucks besteht in der Verwendung einer rotierenden beheizten, nahtlosen, trommelförmigen perforierten Rundschablone, die über eine Düse mit der Haftschmelzklebemasse beschickt wird. Eine speziell geformte Düsenlippe (Rund- oder Vierkantrakel) preßt die über einen Kanal eingespeiste Selbstklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Mit diesem Druckverfahren kann die Größe und Form der Kalotten definiert festgelegt werden. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Träger, der mit größerem Durchmesser und größerer Kalottenhöhe für rauhe oder stark porige Trägermaterialien vorgesehen ist. Die Positionierung der Kalotten auf dem Träger wird durch die in weiten Grenzen variierbare Geometrie des Auftragswerkes, z.B. Gravur- oder Siebgeometrie, definiert festgelegt.

Das Trägermaterial wird bevorzugt mit einer Geschwindigkeit von größer 2 m/min, bevorzugt 20 bis 100 m/min, beschichtet, wobei die Beschichtungstemperatur größer als die Erweichungstemperatur des Klebesystems zu wählen ist.

Die Haftschmelzklebemasse kann mit einem Flächengewicht von größer 15 g/m², bevorzugt zwischen 90 g/m² und 400 g/m², ganz besonders bevorzugt zwischen 130 g/m² und 300 g/m², auf dem Trägermaterial aufgetragen werden.

Der prozentuale Anteil, der mit der Haftschmelzklebemasse beschichteten Fläche sollte mindestens 20% betragen und kann bis zu ca. 95% reichen, für spezielle Produkte bevorzugt 40% bis 60% sowie 70% bis 95%. Dieses kann gegebenenfalls durch Mehrfachapplikation erreicht werden, wobei gegebenenfalls auch Klebemassen mit unterschiedlichen Eigenschaften eingesetzt werden können.

Je nach Trägermaterial und dessen Temperaturempfindlichkeit kann die Selbstklebeschicht direkt aufgetragen oder zuerst auf einen Hilfsträger aufgebracht und dann auf den endgültigen Träger transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Nach der Beschichtung wird das Trägermaterial üblicherweise auf der Klebstoffseite mit einem klebstoffabweisenden Trägermaterial, wie silikonisiertes Papier, abgedeckt. Anschließend werden die Pflaster in der gewünschten Größe ausgestanzt und gegebenenfalls einzeln eingesiegelt und sterilisiert, bevorzugt durch Gamma-Strahlen.

Die folgenden Beispiele sollen die Erfindung beispielsweise erläutern.

### Beispiel 1

Ein wirkstoffhaltiges Pflaster mit einer Heißschmelzselbstklebemasse, welche einen hyperämisierenden Wirkstoff enthält, und zur Anwendung als Rheumapflaster geeignet ist, wurde wie folgt hergestellt:

Auf ein unelastisches Baumwollgewebe mit einer Höchstzugkraft von mehr als 150 N/cm und einer Höchstzugkraft-Dehnung von weniger als 20% als Trägermaterial wurde eine Klebemasse aus
64 Gew.-% A-B/A-B-A Blockcopolymer, welches aus harten und weichen Segmenten besteht, mit einem Verhältnis von A-B-A zu A-B von 3:7 und einem Styrolgehalt im Polymer von 30 Mol.-% (Kraton G, Shell)
32 Gew.-% Paraffinkohlenwasserstoffwachs (H. B. Fuller)
3,5 Gew.-% Kohlenwasserstoffharz (Escorez, Esso)
0,3 Gew.-% Alterungsschutzmittel (Irganox, Ciba-Geigy)
0,2 Gew.-% des hyperamisierenden Wirkstoffs Nonylsäurevanillylamid (Nonivamid)
aufgetragen.

Die eingesetzten Kleberkomponenten wurden in einem Thermomischer bei 175°C 3h homogenisiert. Der Wirkstoff wurde in der Abkühlphase bei 130°C zugegeben und weitere 30 min. im Mischer homogenisiert.

Der Erweichungspunkt der Klebemasse betrug ca. 95°C (DIN 52011), und die Klebemasse zeigte eine Viskosität von 2400 mPas bei 150°C (DIN 53018, Brookfield DV II, Sp 21). Die Glasübergangstemperatur betrug -10°C.

Die Selbstklebemasse wurde mit einer Düse vollflächig auf den Träger aufgebracht. Die direkte Beschichtung erfolgte mit 50 m/min. bei einer Temperatur von 120°C. Das Trägermaterial wurde mit 170 g/m² beschichtet.

Das Pflaster zeigte eine gute Freisetzung des Wirkstoffs. Nach 24-stündiger in vitro-Anwendung an Schweinehaut waren 15% des Wirkstoffs dermal absorbiert. Dieses ist für den schwerflüchtigen Wirkstoff ein vergleichsweise sehr hoher Wert. Der normierte, relative Anteil der dermal adsorbierten Menge teilt sich wie folgt auf:
9,2% in der Hornschicht
26,6% in der Epidermis
64,2% in der Dermis
0,0% Rezeptorphase

### Beispiel 2

Ein wirkstoffhaltiges Pflaster mit einem Trägermaterial wie im Beispiel 1 wurde wie folgt hergestellt:
Auf das Trägermarterial wurde eine Klebemasse aus
50-Gew.-% A-B/A-B-A Blockcopolymer, welches aus harten und weichen Segmenten besteht, mit einem Diblockanteil von 40% und einem Styrolgehalt im Polymer von 17 Mol-% (Kraton D, Shell)
44 Gew.-% Paraffinkohlenwasserstoffharz (Escorez, Esso)
3,5 Gew.-% Kohlenwasserstoffharz (Kaydol, Witco)
1,5 Gew.-% Alterungsschutzmittel (Irganox 1010, Ciba-Geigy)
1,0 Gew.-% des hyperämisierenden Wirkstoffs Nonivamid
aufgetragen.

Die eingesetzten Kleberkomponenten wurden in einem Thermomischer bei 185°C 2,5 h homogenisiert. Der Wirkstoff wurde in der Abkühlphase bei 110°C zugegeben und weitere 45 min im Mischer homogenisiert.

Der Erweichungspunkt der Klebemasse betrug ca. 90°C (DIN 52011), und die Klebemasse zeigte eine Viskosität von 1800 mPas bei 175°C (DIN 53018, Brookfield DV II, Sp 21). Die Glasübergangstemperatur betrug -5°C.

Die Selbstklebemasse wurde imThermosiebdruckverfahren auf den Träger aufgebracht. Hierbei wurde ein 14 Mesh-Sieb der Fa. Stork mit einem Durchlaß von 40% verwendet. Die direkte Beschichtung erfolgte mit 50 m/min. bei einer Temperatur von 135°C. Der Masseauftrag betrug 225 g/m². Die Luftdurchlässigkeit des Pflasters lag bei 55 cm³cm²s.

Das so hergestellte Pflastermaterial zeigte eine sehr gute Freisetzungsrate des Wirkstoffs. Nach 24-stündiger in vitro-Anwendung an Schweinehaut waren 17% des Wirkstoffs dermal absorbiert. Der normierte, relative Anteil der dermal adsorbierten Menge teilt sich wie folgt auf:
12% in der Hornschicht
27% in der Epidermis
61% in der Dermis
0,0% Rezeptorphase

### Beispiel 3 (nicht erfindungsgemäß)

Ein wirkstoffhaltiges Pflaster mit einem Trägermaterial wie in Beispiel 1 wurde wie folgt hergestellt, dabei wurde eine Selbstklebemasse eingesetzt basierend auf einem radikalisch-polymerisierten Acrylatcopolymeren, welches sich aus 70% 2-Ethylhexylacrylat, 20% Butylacrylat und 10% Acrylsäure zusammensetzt. Die Komponenten wurden in einem Reaktor in Lösung bei 70°C zur Reaktion gebracht. Das Lösemittel wurde nach dem Erreichen des Endpunktes entfernt. Die Klebemasse hatte nach der Aufkonzentration einen Feststoffanteil von ca. 99%. In den Feststoffanteil wurden 3% Nonivamid als Wirkstoff in der Homogenisierungsphase eingearbeitet. Der Wirkstoff wurde bei 150°C zugegeben und weitere 90 min. homogenisiert. Die Klebemasse zeigte eine Viskosität von 150 Pas bei 160°C (DIN 53018, Brookfield DV II, Sp. 21). Die Glasübergangstemperatur betrug -30°C.

Die Selbstklebemasse wurde mit einer Düse vollflächig auf den Träger appliziert. Die direkte Beschichtung erfolgte mit 15 m/min. bei einer Temperatur von 165°C. Das Trägermaterial wurde mit 300 g/m² beschichtet.

Das so hergestellte Pflastermaterial zeigte eine vergleichsweise gute Freisetzung des Wirkstoffs. Nach 24-stündiger in vitro-Anwendung an Schweinehaut waren 9% des Wirkstoffs dermal absorbiert. Der normierte, relative Anteil der dermal adsorbierten Menge teilt sich wie folgt auf:
11% in der Hornschicht
32% in der Epidermis
57% in der Dermis
0,0% Rezeptorphase

### Beispiel 4

Ein wirkstoffhaltiges Pflaster mit einem Trägermaterial wie in Beispiel 1 wurde wie folgt hergestellt:
Auf das Trägermaterial wurde eine Klebemasse aus
64-Gew.-% A-B/A-B-A Blockcopolymer, welches aus harten und weichen Segmenten besteht, mit einem Verhältnis von A-B-A zu A-B von 1,85 und einem Styrolgehalt im Polymer von 13 Mol-% (Kraton G, Shell)
26 Gew.-% Paraffinkohlenwasserstoffharz (Escorez 5300, Esso Chemie)
4 Gew.-% Paraffinöle und -wachse (Shell)
0,5 Gew.-% Alterungsschutzmittel (Irganox 1010, Ciba-Geigy)
4,0 Gew.-% des hyperämisierenden Wirkstoffs Capsicumextrakt, entsprechend
1,4% Capsaicinoiden (als Capsaicin berechnet)
aufgetragen.

Die Kleberkomponenten wurden in einem Thermomischer bei 175°C 4 h homogenisiert. Der Wirkstoff wurde in der Abkühlphase bei 130°C zugegeben und weitere 20 min. im Mischer homogenisiert. Die erreichte Homogenität war auf das Soll bezogen größer 90%.

Der Erweichungspunkt der Klebemasse betrug ca. 90°C (DIN 52011), sie zeigte eine Viskosität von 2200 mPas bei 175°C (DIN 53018, Brookfield DV II, Sp 21).

Die Selbstklebemasse wurde mit einer Düse vollflächig auf den Träger aufgebracht. Die direkte Beschichtung erfolgte mit 50 m/min. bei einer Temperatur von 120°C. Das Trägermaterial wurde mit 120 g/m² beschichtet.

Das so hergestellte Pflastermaterial zeigte eine sehr gute Freisetzung des Wirkstoffs. Nach 6-stündiger Anwendung waren 52% der gesamtverfügbaren Menge freigesetzt worden.

### Beispiel 5

Ein wirkstoffhaltiges Pflaster mit einem Trägermaterial wie in Beispiel 1 wurde wie folgt hergestellt:
Auf das Trägermaterial wurde eine Klebemasse aus
60-Gew.-% A-B/A-B-A Blockcopolymer, welches aus harten und weichen Segmenten besteht, mit einem Verhältnis von A-B-A zu A-B von 1,85 und einem Styrolgehalt im Polymer von 13 Mol-% (Kraton G, Shell)
30 Gew.-% Paraffinkohlenwasserstoffharz (Escorez 5300, Esso Chemie)
7 Gew.-% Paraffinöle und -wachse (Shell / H.B. Fuller)
0,5 Gew.-% Alterungsschutzmittel (Irganox 1010, Ciba-Geigy)
2,5 Gew.-% des hyperämisierenden Wirkstoffs Benzylnicotinat
aufgetragen.

Die eingesetzten Kleberkomponenten wurden in einem Thermomischer bei 175°C 4 h homogenisiert. Der Wirkstoff wurde in der Abkühlphase bei 100°C zugegeben und weitere 20 min. im Mischer homogenisiert. Die erreichte Homogenität war auf das Soll bezogen größer 90%.

Der Erweichungspunkt dieser Klebemasse betrug ca. 92°C (DIN 52011), und die Klebemasse zeigte eine Viskosität von 2100 mPas bei 175°C (DIN 53018, Brookfield DV II, Sp 21).

Die Selbsklebemasse wurde im Thermosiebdruckverfahren auf den Träger aufgebracht. Hierbei wurde ein 14 Mesh-Sieb der Fa. Stork mit einem Durchlaß von 40% verwendet. Die direkte Beschichtung erfolgte mit 50 m/min. bei einer Temperatur von 135°C. Der Masseauftrag betrug 225 g/m². Die Luftdurchlässigkeit des Pflasters lag bei 55 cm³/(cm²s).

Das so hergestellte Pflastermaterial zeigt eine sehr gute Freisetzung des Wirkstoffs. Nach 6-stündiger Anwendung waren 31% der verfügbaren Menge freigesetzt worden.

### Beispiel 6

Ein wirkstoffhaltiges Pflaster mit einem Trägermaterial wie in Beispiel 1 wurde wie folgt hergestellt:
Auf das Trägermaterial wurde eine Klebemasse aus
50-Gew.-% A-B/A-B-A Blockcopolymer, welches aus harten und weichen Segmenten besteht, mit einem Verhältnis von A-B-A zu A-B von 1,85 und einem Styrolgehalt im Polymer von 13 Mol.-%. (Kraton G, Shell)
35 Gew.-% Paraffinkohlenwasserstoffharz (Escorez 5300, Esso Chemie)
7 Gew.-% Paraffinöle (Shell)
0,5 Gew.-% Alterungsschutzmittel (Irganox 1010, Ciba-Geigy)
10 Gew.-% des hyperämisierenden Wirkstoffs Propylnicotinat
aufgetragen.

Die eingesetzten Kleberkomponenten wurden in einem Thermomischer bei 175°C 4 h homogenisiert. Der Wirkstoff wurde in der Abkühlphase bei 100°C zugegeben und weitere 120 min. im Thermokneter homogenisiert. Die erreichte Homogenität war auf das Soll bezogen größer 90%.

Der Erweichungspunkt der Klebemasse betrug ca. 82°C (DIN 52011), und die Klebemasse zeigte eine Viskosität von 2800 mPas bei 175°C (DIN 53018, Brookfield DV II, Sp 21).

Die Selbstklebemasse wurde im Thermosiebdruckverfahren auf den Träger appliziert. Hierbei wurde ein 14 Mesh-Sieb der Fa. Stork mit einem Durchlaß von 40% verwendet. Die direkte Beschichtung erfolgte mit 10 m/min. bei einer Temperatur von 135°C. Der Masseauftrag betrug 200 g/m². Die Luftdurchlässigkeit des Pflasters lag bei 50 cm³/(cm²s).

Das so hergestellte Pflastermaterial zeigte eine gute Freisetzung des Wirkstoffs und wie die Pflaster gemäß den anderen Beispielen gute Klebeeigenschaften auch bei dem vergleichsweise hohen Wirkstoffanteil.

## Patentansprüche

1. Wirkstoffhaltige Pflaster mit einem Trägermaterial und einer darauf aufgebrachten Heißschmelzselbstklebemasse, **dadurch gekennzeichnet, dass** die Heißschmelzselbstklebemasse auf A-B/A-B-A- Blockcopolymeren aufgebaut ist und mindestens einen hyperämisierenden Wirkstoff enthält.

2. Wirkstoffhaltige Pflaster gemäß Anspruch 1, **dadurch gekennzeichnet, daß** die Klebemasse den hyperämisierenden Wirkstoff in einer Menge von 0,01 bis 20 Gew.-% enthält.

3. Wirkstoffhaltige Pflaster gemäß Anspruch 2, **dadurch gekennzeichnet, daß** die Klebemasse den hyperämisierenden Wirkstoff in einer Menge von 0,1 bis 10 Gew.-% enthält.

4. Wirkstoffhaltige Pflaster gemäß Anspruch 1, 2 oder 3, **dadurch gekennzeichnet, daß** hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, eingesetzt werden.

5. Wirkstoffhaltige Pflaster gemäß einem der vorhergehenden Ansprüche, **dadurch gekennzeichnet, dass** die Phase A der Heißschmelzselbstklebemasse Polystyrol oder dessen Derivate und Phase B Ethylen, Propylen, Butylen, Butadien, Isopren oder deren Mischungen, hierbei besonders bevorzugt Ethylen und Butylen oder deren Mischungen, sind.

6. Wirkstoffhaltige Pflaster gemäß Anspruch 5, **dadurch gekennzeichnet, dass** die auf A-B/A-B-A- Blockcopolymeren aufgebaute Heißschmelzselbstklebemasse einen Anteil an A-B-Blockcapolymeren von weniger als 80 Gew.-% besitzt.

7. Wirkstoffhaltige Pflaster gemäß Anspruch 5 oder 6, **dadurch gekennzeichnet, dass** der gesamte Styrolanteil im Polymer niedriger als 35 Gew.-%, bevorzugt 5 Gew.-% bis 30 Gew.-%, ist.

8. Wirkstoffhaltige Pflaster gemäß einem der vorhergehenden Ansprüche, **dadurch gekennzeichnet, dass** die Heißschmelzselbstklebemasse enthält
| | |
|---|---|
| 10 bis 90 Gew.-% | A-B/A-B-A- Blockcopolymere, |
| 5 bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher |
| weniger als 15 Gew.-% | Additive |
| weniger als 5 Gew.-% | Stabilisatoren |
| 0,1- 10 Gew.-% | Wirkstoff oder Wirkstoffe. |

9. Wirkstoffhaltige Pflaster gemäß einem der vorhergehenden Ansprüche, **dadurch gekennzeichnet, dass** die wirkstoffhaltige Heißschmelzselbstklebemasse vollflächig oder flächenmäßig begrenzt aufgetragen ist.

10. Wirkstoffhaltige Pflaster gemäß Anspruch 9, **dadurch gekennzeichnet, dass** die Heißschmelzselbstklebemasse flächenmäßig begrenzt im Siebdruck aufgetragen ist mit einer Flächendeckung von 20 - 95%.

11. Wirkstoffhaltige Pflaster gemäß einem der vorhergehenden Ansprüche, **dadurch gekennzeichnet, dass** die Heißschmelzselbstklebemasse auf einem A-B/A-B-A Blockcopolymeren aus Styrol, Äthylen und Butylen aufgebaut ist mit einem Styrolanteil von weniger als 35 Gew.-%.

12. Wirkstoffhaltige Pflaster gemäß Anspruch 11, **dadurch gekennzeichnet, dass** die Heißschmelzselbstklebemasse etwa 0,1 bis 10 Gew.-%, bevorzugt etwa 0,2 Gew.-% Nonivamid enthält.

13. Wirkstoffhaltige Pflaster gemäß einem der vorhergehenden Ansprüche, **dadurch gekennzeichnet, dass** Polystyrol bis zu einem Anteil von 20 Gew.% in der Phase B gleichmäßig bzw. ungleichmäßig verteilt ist.

## Claims

1. Plasters containing active substance, comprising a backing material and, applied thereon, a hot-melt self-adhesive composition, **characterized in that** the hot-melt self-adhesive composition is based on A-B/A-B-A block copolymers and comprises at least one hyperaemic active substance.

2. Plasters containing active substance according to Claim 1, **characterized in that** the adhesive composition comprises the hyperaemic active substance in an amount of from 0.01 to 20% by weight.

3. Plasters containing active substance according to Claim 2, **characterized in that** the adhesive composition comprises the hyperaemic active substance in an amount of from 0.1 to 10% by weight.

4. Plasters containing active substance according to Claim 1, 2 or 3, **characterized in that** hyperaemic active substances such as natural active substances of cayenne pepper or synthetic active substances such as nonivamide and nicotinic acid derivatives, preferably bencyl [sic] nicotinate or propyl nicotinate, are employed.

5. Plasters containing active substance according to one of the preceding claims, **characterized in that** phase A of the hot-melt self-adhesive composition is polystyrene or derivatives thereof and phase B is ethylene, propylene, butylene, butadiene, isoprene or mixtures thereof, among which particular preference is given to ethylene and butylene or mixtures thereof.

6. Plasters containing active substance according to Claim 5, **characterized in that** the hot-melt self-adhesive composition based on A-B/A-B-A block copolymers has a proportion of A-B block copolymers of less than 80% by weight.

7. Plasters containing active substance according to Claim 5 or 6, **characterized in that** the overall content of styrene in the polymer is less than 35% by weight, preferably from 5% by weight to 30% by weight.

8. Plasters containing active substance according to one of the preceding claims, **characterized in that** the hot-melt self-adhesive composition comprises
| | |
|---|---|
| from 10 to 90% by weight | of A-B/A-B-A block copolymers, |
| from 5 to 80% by weight | of tackifiers, such as oils, waxes, resins and/or mixtures thereof, preferably mixtures of resins and oils, |
| less than 60% by weight | of plasticizers |
| less than 15% by weight | of additives |
| less than 5% by weight | of stabilizers |
| 0.1 - 10% by weight | of active substance or substances. |

9. Plasters containing active substance according to one of the preceding claims, **characterized in that** the hot-melt self-adhesive composition containing active substance is applied over the full area or with areal restriction.

10. Plasters containing active substance according to Claim 9, **characterized in that** the hot-melt self-adhesive composition is applied, with areal restriction, by screen printing, with an areal coverage of 20-95%.

11. Plasters containing active substance according to one of the preceding claims, **characterized in that** the hot-melt self-adhesive composition is based on an A-B/A-B-A block copolymer of styrene, ethylene and butylene with a styrene content of less than 35% by weight.

12. Plasters containing active substance according to Claim 11, **characterized in that** the hot-melt self-adhesive composition contains from about 0.1 to 10% by weight, preferably about 0.2% by weight, of nonivamide.

13. Plasters containing active substance according to one of the preceding claims, **characterized in that** polystyrene is distributed uniformly or nonuniformly in phase B in a proportion of up to 20%.

## Revendications

1. Pansement contenant des agents actifs, ayant un matériau de support et une masse auto-adhésive thermofusible qui y est appliquée, **caractérisé en ce que** la masse auto-adhésive thermofusible est constituée de copolymères séquencés A-B/A-B-A et **en ce qu'**elle contient au moins un agent actif favorisant la circulation sanguine.

2. Pansement contenant des agents actifs selon la revendication 1, **caractérisé en ce que** la masse adhésive contient l'agent actif favorisant la circulation sanguine dans une quantité de 0,01 à 20% en poids.

3. Pansement contenant des agents actifs selon la revendication 2, **caractérisé en ce que** la masse adhésive contient l'agent actif favorisant la circulation sanguine dans une quantité de 0,1 à 10% en poids.

4. Pansement contenant des agents actifs selon la revendication 1, 2, ou 3, **caractérisé en ce que** l'on utilise des agents favorisant la circulation sanguine, comme les agents actifs naturels du poivre de Cayenne ou des agents actifs synthétiques comme le produit Nonivamid, les dérivés de l'acide nicotinique, de préférence, le nicotinate de benzyle ou le nicotinate de propyle.

5. Pansement contenant des agents actifs selon l'une quelconque des revendications précédentes, **caractérisé en ce que** la phase A de la masse auto-adhésive thermofusible est le polystyrène ou ses dérivés et **en ce que** la phase B de la masse auto-adhésive thermofusible est l'éthylène, le propylène, le butylène, le butadiène, l'isoprène ou leurs mélanges, en l'occurrence en particulier de préférence, l'éthylène et le butylène ou leurs mélanges.

6. Pansement contenant des agents actifs selon la revendication 5, **caractérisé en ce que** la masse auto-adhésive thermofusible constituée de copolymères séquencés A-B/A-B-A possède une proportion de copolymères séquencés A-B de moins de 80% en poids.

7. Pansement contenant des agents actifs selon la revendication 5 ou 6, **caractérisé en ce que** la proportion de styrène globale dans le polymère est inférieure à 35% en poids, étant, de préférence, de 5% en poids jusqu'à 30% en poids.

8. Pansement contenant des agents actifs selon l'une quelconque des revendications précédentes, **caractérisé en ce que** la masse auto-adhésive thermofusible contient
de 10 à 90% en poids de copolymères séquencés A-B/A-B-A,
de 5 à 80% en poids d'agents collants, comme des huiles, des cires, des résines et/ou leurs mélanges, de préférence, des mélanges de résines et d'huiles,
moins de 60% en poids de plastifiants,
moins de 15% en poids d'additifs,
moins de 5% en poids d'agents de stabilisation,
de 0,1 - 10% en poids d'agent actif ou d'agents actifs.

9. Pansement contenant des agents actifs selon l'une quelconque des revendications précédentes, **caractérisé en ce que** la masse auto-adhésive thermofusible contenant des agents actifs est appliquée sur toute la surface ou d'une manière limitée par rapport à la surface totale.

10. Pansement contenant des agents actifs selon la revendication 9, **caractérisé en ce que** la masse auto-adhésive thermofusible est appliquée par sérigraphie d'une manière limitée par rapport à la surface totale, avec un degré de recouvrement de 20-95%.

11. Pansement contenant des agents actifs selon l'une quelconque des revendications précédentes, **caractérisé en ce que** la masse auto-adhésive thermofusible est constituée d'un copolymère séquencé A-B/A-B-A fait de styrène, d'éthylène et de butylène, avec une proportion de styrène de moins de 35% en poids.

12. Pansement contenant des agents actifs selon la revendication 11, **caractérisé en ce que** la masse auto-adhésive thermofusible contient environ de 0,1 à 10% en poids, de préférence, environ 0,2% en poids du produit Nonivamid.

13. Pansement contenant des agents actifs selon l'une quelconque des revendications précédentes, **caractérisé en ce que** le polystyrène est réparti, de manière uniforme ou de manière non uniforme, jusqu'à raison d'une proportion de 20% en poids dans la phase B.
